# EUROPEAN PATENT SPECIFICATION

(11) **EP 1 621 546 B1**
(45) Date of publication and mention of the grant of the patent: **29.10.2008**
(21) Application number: 05076751.6
(22) Date of filing: 28.07.2005
(51) Int. Cl.: C07K 5/08, C07K 5/10, C07K 7/02, C07K 5/02, C07K 16/06, G01N 33/68

(54) **Peptide ligands specific to immonoglobulins**
Peptidische Ligande die eine spezifische Bindungsfähigkeit für Immunoglobuline zeigen
Ligands peptidiques avec affinité spécifique pour les immunoglobulines

(30) Priority: 30.07.2004 IT MI20041569
(43) Date of publication of application: 01.02.2006
(73) Proprietor: Tecnogen S.p.A., 81015 Piana di Monte Verna (Caserta) (IT)
(72) Inventor: Verdoliva, Antonio, 80143 Napoli (IT); Bellofiore, Piero, 81022 Casagiove CE (IT); Cassani, Giovanni, 27100 Pavia (IT); Marasco, Daniela, 80147 Napoli (IT); Pedone, Carlo, 80121 Napoli (IT); Monti, Simona, 80122 Napoli (IT); Ruvo, Menotti, 83058 Trevico AV (IT)
(74) Representative: Marchi, Massimo

(56) References cited:
- EP-A- 0 497 585
- EP-A- 1 403 281
- EP-A- 1 479 764
- WO-A-00/58005
- WO-A-02/059340
- WO-A-03/084477
- CA-A- 2 290 722
- JP-A- 2004 081 178
- RU-C- 2 206 573
- US-A1- 2003 113 322
- US-A1- 2004 039 543
- US-B1- 6 469 153
- JOURNAL OF BIOCHEMICAL AND BIOPHYSICAL METHODS, vol. 49, 2001, pages 481-490, XP002325189 NL AMSTERDAM
- DATABASE CA [Online] CHEMICAL ABSTRACTS SERVICE, COLUMBUS, OHIO, US; FASSINA, GIORGIO: "Combinatorial technologies in affinity chromatography: Discovery of a synthetic ligand for antibody purification" XP002325190 retrieved from STN Database accession no. 136:400120 & AMERICAN BIOTECHNOLOGY LABORATORY , 19(12), 46, 48 CODEN: ABLAEY; ISSN: 0749-3223, 2001,

## Description

The present invention relates to novel synthetic peptides capable of binding themselves non-covalently to immunoglobulins, a method for preparing thereof and the use thereof in the chromatographic and analytical field.

The immunoglobulins are of particular importance in the diagnostic and therapeutic field. In the first case they are mainly used as reagents in the identification and quantification of molecules present in biological fluids. In the second case, in turn, they are used as agents capable of binding selectively biological molecules involved in physiological processes which are therapeutically relevant. Given their importance, it follows that their production and, especially, their purification are particularly relevant in the industrial field.

The immunoglobulins, also known as "antibodies", are generally obtained from animal sera or from suitable cell lines cultures. Their purification is usually carried out by procedures that comprise precipitation with inorganic salts or organic solvents, such as ammonium sulphate and ethanol, chromatographic techniques, such as ion exchange, gel filtration, hydroxyapatite, and electrophoretic techniques. In general, however, these methods are not readily reproducible on an industrial scale and always involve a compromise between yield and purity of the final product.

At present, affinity chromatography is the most widely used procedure for the purification of antibodies from biological fluids, and, even though some ligands or synthetic matrices alternative to Protein A have already been proposed (EP-A-0 752 425; Fassina, G. et al. (1996), J Mol Recognit. 9 (5-6), 564-569; Li, R. et al. (1998), Nat Biotechnol. Feb.;16(2):190-5; Pitiot, O. et al. (2001), J Chromatogr B Biomed Sci. Appl. 758(2):173-82; Verdoliva, A. et al. (2002). J. Immunol. Methods, 271 (1-2), 77-88), the method most commonly used for antibody purification is still chromatography on columns obtained by immobilizing Protein A.

Protein A, extracted from *Staphylococcus aureus*, is able to bind specifically and with high affinity to the constant region of the immunoglobulins, thus allowing a quick and selective purification of antibodies (Fuglistaller, P. (1989), J. Immunol. Methods, 124, 171; Godfrey, M.A. et al. (1993), J. Immunol. Methods 160, 97; Siodahl, J., (1977), Eur. J. Biochem., 78, 471-490).

However, the wide-scale application of Protein A obtained from microorganisms or from genetically modified bacteria has many limits. Indeed, its preparation involves complex and expensive steps that usually require further analytical controls for checking the presence of contaminants that may impair the quality of the purified product (viruses, pyrogens, DNA etc.). Furthermore, Protein A is not very stable in denaturing conditions or in the presence of agents used for the removal of biological contaminants such as viruses or fragments of nucleic acids.

Therefore there is still a great need for synthetic compounds that are capable of binding immunoglobulins non-covalently and that, thank to their synthetic nature, are devoid of biological contaminants and are stable even in drastic experimental conditions.

Synthetic ligands for antibodies have been described in American Biotechnology Laboratory (2001), 19(12), pages 46-48.

It has been found that these features are owned by the following peptide sequence:

X₁-X₂-His-X₃-CO (S)

where:
- X₁: is H or an amino acid residue of cysteine (Cys) having L or D configuration,
- X₂: is an amino acid residue of tyrosine (Tyr) or of histidine (His) having L or D configuration,
- X₃: is an amino acid residue of His or Tyr having L or D configuration,
- His: is an amino acid residue of histidine having L or D configuration, provided, however, that X₂ is Tyr when X₃ is His and X₂ is His when X₃ is Tyr.

Preferably all the amino acids of sequence (S) have D configuration.

Therefore it is a first object of the present invention to provide a compound of formula:

(X₁-X₂-His-X₃-CO)ₙ-R (I)

where;
- X₁: is H or an amino acid residue of cysteine (Cys) having L or D configuration, wherein each pair of amino acid residues of Cys optionally present as X₁, can be in the form of a cystine residue owing to an -S-S- bridge formed by the -SH functions of two Cys residues,
- X₂: is an amino acid residue of tyrosine (Tyr) or of histidine (His) having L or D configuration,
- X₃: is an amino acid residue of His or Tyr having L or D configuration,
provided, however, that X₂ is Tyr when X₃ is His and X₂ is His when X₃ is Tyr,
- His: is an amino acid residue of histidine having L or D configuration,
- n: is an integer of from 2 to 4,
- R: is a group capable of forming a dimer, a trimer and, respectively, a tetramer, comprising from 1 to 3 amino acid residues of lys.

Advantageously, the N-terminal group or groups of Compound I are acylated by acylating groups capable of neutralizing their nucleophilicity/basicity and/or of non-covalently immobilizing said Compound I on a solid support.

Examples of said acylating groups are
- natural amino acids, such as: L-tryptophan, D-tryptophan, L-tyrosine, D-tyrosine;
- synthetic amino acids, such as: L-naphthylalanine, cyclohexylalanine, aminonorbomyl carboxylate, L-N-methyltryptophan, L-N-methyltyrosine, N-methyl-α-naphthylalanine, D-N-methyltryptophan, D-N-methoyltyrosine, N-benzylglycine, N-(3-indolylethyl)glycine, N-(p-hydroxyphenyl-ethyl)glycine, N-cyclopropyl-glycine, N-cyclobutyl-glycine, N-cyclo-hexyl-glycine, N-cyclo-opta-glycine, N-cyclooctylglycine, N-cyclodecylglycine, N-cycloundecylglycines, N-cyclododecylglycines, N-(2,2-diphenylethyl)glycine, N-(3,3-diphenyl-propyl)glycine, N-(N-(2,2-diphenylethyl)carbamyl-methyl)-glycine, N-(N-(3,3-diphenyl-propyl)carbamylmethyl)glycine, 1-carboxy-1-(2,2-diphenyl-ethylamino)-cyclopropane;
- carboxylic acids and their derivatives, such as: naphthylacetic, benzoic, 4-methoxybenzoic, 2,4-dichlorobenzoic, 2-nitrobenzoic, 4-chlorobenzoic, phenylacetic, diphenylacetic, (R)-2-phenylpropanoic, (S)-2-phenylpropanoic, (S)-3-phenylpropanoic, (R)-3-phenylpropanoic, (R,S)-2-methyl,3-phenylbutanoic, (S,R)-2-methyl,3-phenylbutanoic, (R,R)-2-methyl,3-phenylbutanoic, (S,S)-2-methyl,3-phenylbutanoic, (R,S)-2,3-diphenylpentanoic, (R,R)-2,3-diphenylpentanoic, (S,S)-2,3-diphenylpentanoic, and (S,R)-2,3-diphenylpentanoic acid, 9-fluorenyl-methoxycarbonyl chloride, carbobenzyloxy chloride, 2-chloro carbobenzyloxy chloride.

Examples of groups capable of non-covalently immobilizing a peptide on a solid support are fluorenyl-methoxycarbonyl (Fmoc), carboxybenzyl (Cbz) (Bodansky, M., Bodansky, A., (1995), The practice of peptide synthesis (2nd edn.), Springer Verlag, Berlin; Fields, G.B. (ed.), (1997), Solid-Phase Peptide Synthesis in Methods in ENZYMOLOGY, Vol. 289, Academic Press, San Diego).

In this description and in the claims, the term "solid support" is intended to mean those solid bodies that are well-known to the person skilled in the art for immobilizing thereon peptides for analytical and/or diagnostic purposes. Usually said solid bodies are in the form of shaped bodies such as, for example, spherules and granules, made of synthetic polymers such as PVC, polystyrene and polyethylene.

Typical examples of said shaped bodies are ELISA plates, which have wells, on the surfaces of which the Compound I of the present invention can be immobilized.

The terms dimer, trimer and tetramer indicate a peptide comprising 2, 3 or, respectively, 4 (S) sequences.

A typical example of a group R capable of forming a dimer (n = 2) is a lysine residue (Lys). The dimer thus obtained has the following formula: 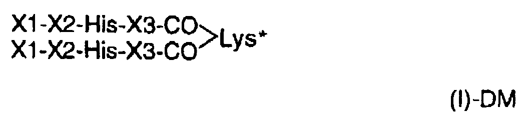 where
- X₁, X₂ and X₃: have the meanings stated above, and
- Lys*: indicates a lysine residue that still has a free carboxyl function or a carboxy-amide function.

A typical example of a group R capable of forming a trimer (n = 3) is a lysyl-lysine (Lys-Lys) dipeptide. The trimer thus obtained has the following formula: 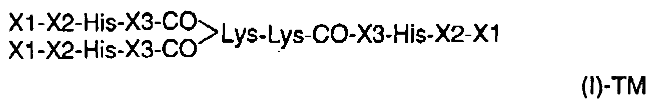 where
X₁, X₂, and X₃ have the meanings stated above.

A typical example of a group R capable of forming a tetramer (n = 4) is a branched tripeptide of formula Lys-Lys-(εLys). The tetramer thus obtained has the following formula: 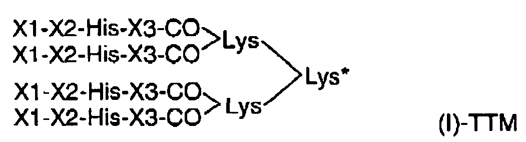 where X₁, X₂, X₃ and Lys* have the meanings stated above.

For the sake of simplicity, the aforesaid tetramer can also be written in the following pseudo-linear form:

(X₁-X₂-His-X₃-CO)₄-Lys₂-Lys*

Group R may also be bound to a group capable of immobilizing a peptide on a solid support. Moreover, or alternatively, group R can be bound to a molecule having useful properties in the analytical field such as, for example, biotin and fluorescein.

In a preferred embodiment, the group R is an amino acid residue of lysine that comprises a reactive free carboxyl or amino group, whose reactivity is utilized for binding said group R to a group bearing (i) at least one group capable of immobilizing Compound I on a solid support and/or (ii) at least one molecule having useful properties in the analytical field.

Typically, the group R is bound to a group of formula:

-HN-(Gly)ₘ-Lys-(ε-R₃)-CO-R₄ (Z)

where
- m: is an integer of from 1 to 5, and
- R₃: is a hydrophobic group capable of immobilizing a peptide by adsorption on a solid support or a molecule having useful properties in the analytical field,
- R₄: is OH or NH₂.

A typical example of a dimer of formula (I) bearing a hydrophobic group (Cbz) capable of immobilizing the dimer by adsorption on a solid support has the following formula:

(H₂N-Cys-Tyr-His-His-CO)₂-Lys-Gly₃-Lys-(ε-Cbz)-COOH (IA)

where
the pair of Cys amino acid residues can be in the form of a cystine residue owing to an -S-S- bridge formed by the -SH functions of the two Cys residues, and
one or both the N-terminal groups (NH₂) are acylated by acylating groups capable of neutralizing their nucleophilicity and/or basicity or of immobilizing a peptide on a solid support.

Compounds (I) of the invention can be prepared easily in accordance with the usual techniques of liquid-phase or solid-phase preparation of peptides comprising protection, coupling and deprotection of amino acids.

Therefore, another object of the present invention is a method of preparation of a Compound I as defined above according to the solution or solid-phase technique.

Preparation according to the technique of solid-phase synthesis is preferably carried out manually employing simple equipment, such as plastic tubes equipped with filtering diaphragms and stirrers, or by means of automatic synthesizers, an example of which is the model 433A manufactured by Applied Biosystems™ (Foster City, CA, USA).

Advantageously, the preparation is carried out in accordance with usual methods familiar to the person skilled in the art, such as the methods described by Bodansky *I*.*c*. and by Fields *I*.*c*.

Typically, a method of solid-phase synthesis according to the present invention comprises the following steps:
a) coupling a first amino acid in D or L configuration to a labile acidic support for solid-phase peptide synthesis; the functional group of said first amino acid used for anchoring to the resin being such that it can be hydrolysed by treatment with strong acids such as trifluoroacetic acid (TFA), whereas the second functional group is provided with a suitable protection that can be removed after anchoring to the resin. If the first amino acid is trifunctional such as, for example, Cys or Lys, two functions will be protected before anchoring to the resin;
b) optional addition of groups such as biotin or fluorescein on a side function;
c) optional sequential addition of 2-7 amino acid residues, still according to the protection-coupling-deprotection technique, said amino acid groups preferably comprising glycine and/or D- or L-alanine;
d) subsequent sequential coupling of 1 (dimer), 2 (trimer) or 3 (tetramer) residues of D- or L-lysine protected at their α and ε amino functional groups with the same group such as the group Fmoc,
e) sequential coupling of suitable amino acid residues such as D- or L-histidine protected at the N^{τ} imidazole group with a suitable group such as triphenylmethyl (trityl or Trt), or D- or L-tyrosine protected at the phenolic group with a suitable group such as tert-butyl (tBu); a D- or L-histidine with similar Trt protection; a residue of D- or L-histidine protected at the N^{τ} imidazole group with a Trt group, or D- or L-tyrosine protected at the phenolic group with a tBu group; finally a residue of D- or L-cysteine provided with Trt protection on the thyol function; all the amino acid residues also being suitably protected on the α amino function by a suitable removable protecting group such as Fmoc;
f) addition to the N-terminal functions of the last amino acid, of optional protecting groups such as acetyl groups, Fmoc, Cbz;
g) splitting of the various protecting groups of the peptide with the exclusion, optionally, of those on the N-terminal groups and removal thereof from the resin, and
h) optional cyclization of the peptide by formation of a disulphide bridge between the cysteine pairs optionally present on said peptide molecule.

The Compounds of formula (I) of the present invention are able to bind non-covalently at least one immunoglobulin.

Therefore, a further object of the following invention is to provide a method of separating and purifying at least one immunoglobulin that comprises the following steps;
(i) immobilization on a support for affinity chromatography of a compound that is able to bind non-covalently at least one immunoglobulin,
(ii) packing of said support for affinity chromatography in a chromatographic column,
(iii) flushing of said column with a buffer capable of promoting the formation of bonds between immunoglobulin and the immobilized compound,
(iv) loading of said column with a fluid comprising at least one immunoglobulin,
(v) washing of said column with a liquid capable of eluting the impurities without interfering with the bond between the immunoglobulin and the immobilized compound,
(vi) elution of the immunoglobulin adsorbed on the column with a dissociating eluent,
and is characterized in that:
the compound that is capable of binding non-covalently at least one immunoglobulin is a Compound of formula (I) as defined above.

The steps from (i) to (vi) can be carried out according to conventional techniques that are familiar to the person skilled in the art and are widely described in the literature (Narayanan, S.R. (1994), J. of Chromatography, 658, 237-258; Lowe, C.R. (1978), "Laboratory Technique in Biochemistry and Molecular Biology". Work and Burdon, Vol. 7, part 2, Elsevier, N. Holland, Amsterdam).

Preferably, the support for affinity chromatography is of the type pre-activated with an epoxy, azalactone or ester group for direct coupling of peptides and proteins. Typical examples of suitable supports are the resins CH-Sepharose-4B™ and CH-Sepharose-Fast Flow™ from Amersham Biosciences™ (England), activated with an N-hydroxysuccinimide (NHS) ester; the resin Eupergit C30N™ from Rohm and Haas™ (Germany), activated with an epoxy function; the resin 3M Emphaze Biosupport Medium™ from Pierce (USA), activated with a function of the azalactone type.

Step (i) is preferably carried out in the presence of a basic buffer solution where the pH is preferably of from 8.5 to 9.5, and does not comprise compounds provided with free amino functions or other nucleophilic groups.

Step (iii) is preferably carried out with a neutral buffer solution, for example a solution of Bis-Tris, pH 6.5, or a solution of sodium phosphate, pH 7.0.

Step (v) is preferably carried out with a neutral buffer solution of low ionic strength, for example a solution of Bis-Tris 25 mM, pH 6.5, or a solution of sodium phosphate, 50 mM, pH 7.0.

Examples of dissociating eluents suitable for carrying out step (vi) are acidic and basic aqueous solutions.

Typical examples of said solutions are the aqueous solutions of acetic acid 0.1 M pH 3.0, and of sodium bicarbonate 100 mM pH 9.0.

The Compounds of formula (I) of the present invention can also be used in the qualitative and quantitative analysis of immunoglobulins by the ELISA technique.

Therefore, still another object of the present invention is to provide a method for detecting an immunoglobulin or a mixture of immunoglobulins by the ELISA technique comprising the following steps:
(1) immobilization on a microplate for ELISA analysis of a compound capable of binding non-covalently at least one immunoglobulin,
(2) incubation, on the microplate, of a sample comprising the immunoglobulin or immunoglobulins of interest,
(3) detection of the complex formed from the immobilized compound/immunoglobulin,
and is characterized in that:
the compound capable of binding at least one immunoglobulin is a Compound of formula (I) as previously defined.

The analytical detection of immunoglobulins by the ELISA technique is widely described in the literature; see, for example, Johnstone, A.P. and Thorpe, R.C. (1996), *Immunochemistry in Practice,* Third edition. 362 pages. Blackwell Science, Oxford.

Step (1) is preferably carried out with microplates made of a plastic material, for example PVC, having 96 wells which are filled with solutions of sodium bicarbonate 0.1 M, pH 9.0 and comprise variable amounts (0-50 µg/mL) of ligand. After 12-24 hours of incubation at 4ºC or for 1 hour at room temperature, the plates are washed with phosphate buffer and the wells are filled with a 3% solution of blocking agent (bovine albumin, milk powder, etc.) to eliminate any sites of nonspecific interaction.

In step (2), the microplates are washed with phosphate buffer and the respective wells are filled with solutions comprising the immunoglobulins to be analysed. The microplates are then incubated at 20-37ºC for a further 1-18 hours.

Finally, step (4) is carried out by adding, to each well, a solution of secondary antibody (antibody to the immunoglobulins under analysis), preferably derivatized with biotin or with peroxidase.

After 2 hours of incubation the microplates are washed again with phosphate buffer. In the case of biotinylated secondary antibodies, a solution of streptavidin or avidin conjugated to peroxidase is added to each well, allowed to incubate for 2 hours and, after washing with phosphate buffer, a solution of OPD or ABTS is added and the optical density of each well is measured with a suitable microplate reader. In the case of secondary antibody conjugated to peroxidase, the step of development with OPD or ABTS is carried out directly.

This and other features of the present invention will become clearer from the examples that follow and from the appended drawings wherein:
- Fig. 1 shows the chromatogram relating to the purification of immunoglobulins from human serum by affinity chromatography according to Example 3 hereunder;
- Fig. 2 shows the analysis by polyacrylamide gel electrophoresis of the fractions derived from purification of immunoglobulins from raw human serum according to Example 3 hereunder.

Solid-phase synthesis of the Compounds of formula (I) was carried out both using a manual procedure and the automatic synthesizer, model 433 A, software version 1.2, made by Applied Biosystems™ (Foster City, CA, USA), in accordance with the synthesis procedures recommended by the supplier, based on methods that are well described in the literature (Fields *I*.*c*., Bodansky *I.c*.).

In the examples that follow, the abbreviation "Cat. No." stands for "catalogue number".

The examples that follow are intended to better describe this invention without, however, limiting the same.

### EXAMPLE 1

Solid-phase synthesis of Compound IB (wherein X₁ = L-Cys, X₂ = L-Tyr, X₃ = L-His, R = L-Lys-L-Gly) of formula:

(NH₂-L-Cys-L-Tyr-L-His-L-His)₂-L-Lys-L-Gly-OH (IB)

having a cyclic structure wherein the C-terminal regions of two L-Cys-L-Tyr-L-His-L-His sequences are bound to α and ε amino groups of a lysine (Lys) and the two cysteines (Cys) are in the form of a cystine residue owing to an -S-S- bridge formed by the -SH functions of two Cys residues.

Preparation of Compound IB was carried out manually by solid-phase synthesis according to the Fmoc/HOBt/HBTU technique on a scale of 100 µmol using, as solid support, a resin of polystyrene-1% divinylbenzene, 100-200 mesh, derivatized with the p-benzyloxy-benzyl group (substitution 0.75 meq/g) (Novabiochem™, Laufelfingen, Switzerland, Cat. No. 01-64-0014). The amount of resin used was therefore of 133.0 mg.

The resin was washed 3 times with 5 ml of a 3:2 mixture of dichloromethane/dimethylformamide (DCM, LabScan™, Dublin, Ireland. Cat. No. H6508L; DMF, LabScan™, Dublin, Ireland, Cat. No. H33H11X). Meanwhile, 1.0 mmol of Fmoc-Gly-OH (Novabiochem™, Laufelfingen, Switzerland, Cat. No. 04-12-1001) was dissolved in 3.0 ml of a 3:2 mixture of DCM/DMF. 0.5 mL of a freshly prepared 1.0 M solution in NMP (N-methyl-pyrrolidone) of dicyclohexylcarbodiimide (DCC, Sigma-Aldrich™, Milan, Italy, Cat. No. 36651) was added to the solution. The thus obtained solution was stirred for 20 minutes at room temperature and was then filtered to remove the white precipitate of dicyclohexylurea (DCU). After addition of 0.36 ml of a 0.1 M solution of dimethyl aminopyridine (DMAP, Sigma-Aldrich™, Milan, Italy, Cat. No. 39408) in DMF, which acts as catalyst, the mixture was added to the resin and the suspension was stirred for 1 hour. The solution was separated from the resin and was washed once with 5.0 mL of DMF/DCM 2:3 and then 3 more times with 5.0 ml of DMF. After removal of the solvent, the resin was treated with 2.0 mL of acetic anhydride (Sigma-Aldrich™, Milan, Italy, Cat. No. 45830) 2.0 M in DMF, comprising 0.72 mL of 0.1 M solution of DMAP in DMF and 5% of diisopropylethylamine (DIEA, Sigma-Aldrich™, Milan, Italy, Cat. No. 03440).

Removal of the Fmoc group was carried out by treating the resin with 3.0 mL of piperidine solution (Sigma-Aldrich™, Milan, Italy, Cat. No. 80641) in DMF 20:80 (v/v) for 15 minutes at room temperature and then washing 3 times with 3.0 mL of DMF. The reaction yield was evaluated spectrophotometrically by determining the amount of Fmoc-piperidine adduct released in the deprotection solution. For this purpose, the deprotection solution and the washing solvent were combined (total volume 12 mL) and a small aliquot was diluted 100 times with DMF. The concentration of the adduct was determined by reading the absorbance at 301 nm and using a molar extinction coefficient ε₃₀₁ = 7.8 mM⁻¹cm⁻¹. The absorbance of the solution was 0.640, corresponding to 98.4 µmol of glycine bound to the resin.

For coupling the next amino acid, 0.5 mmol of Fmoc-L-Lys(Fmoc)-OH (Novabiochem™, Laufelfingen, Switzerland, Cat. No. 04-12-1085) was dissolved in 1.5 mL of DMF and the the solution was added with 1.0 mL of a 0.5 M solution of HBTU/HOBt (hexafluorophosphate of O-(benzotriazol-1-yl)-N,N,N',N'-tetramethyluronium, HBTU, Sigma-Aldrich™, Milan, Italy, Cat. No. 12804; N-hydroxy-benzotriazole, HOBt, Sigma-Aldrich™, Milan, Italy, Cat. No. 54802) in DMF (corresponding to 0.5 mmol of HBTU and 0.5 mmol of HOBt) and 0.5 mL of DIEA 2.0 M in N-methylpyrrolidone (NMP, 1.0 mmol) and allowed to incubate for 3 minutes. The solution of the activated amino acid was transferred onto the resin and stirred for 30 minutes at room temperature, finally it was washed 3 times with 3.0 mL of DMF. Deprotection of the two Fmoc groups of the lysine was carried out by treating the resin with 5.0 mL of 20% piperidine in DMF for 15 minutes and washing 3 times with 5.0 mL of DMF. The deprotection solution and the solvent used for the washings were mixed together and detection of Fmoc as described previously was carried out on the resulting mixture. Spectrophotometric detection gave a value of 196.4 µmol (yield 99.8%).

For adding the first two histidines, 1.0 mmol of Fmoc-L-His(Trt)-OH (Novabiochem™, Laufelfingen, Switzerland, Cat. No. 04-12-1065) was dissolved in 3.0 mL of DMF and activated with 1.0 mmol (2.0 mL of a 0.5 M solution) of HBTU/HOBt and 2.0 mmol of DIEA (1.0 mL of a 2.0 M solution in NMP) for 3 minutes, then the solution was transferred to the resin and allowed to react at room temperature for 30 minutes. The resin was washed 3 times with 5.0 mL of DMF and then treated as described above for 15 minutes with 5.0 mL of 20% piperidine in DMF determining the amount of piperidine-Fmoc adduct released in the deprotection solution. Analysis gave a value of 196.1 µmol (yield 99.8%).

The second histidine was coupled by working in exactly the same way, pre-activating 1.0 mmol of Fmoc-L-His(Trt)-OH (Novabiochem™, Laufelfingen, Switzerland, Cat. No. 04-12-1065) with HBTU/HOBt/DIEA and allowing reacting for 30 minutes. Analysis of the adduct after deprotection (15 minutes with 5.0 mL of 20% piperidine/DMF) gave an experimental value of 194.5 µmol (yield 99.2%).

For coupling the two tyrosines, 1.0 mmol of Fmoc-L-Tyr(tBu)-OH (Novabiochem™, Laufelfingen, Switzerland, Cat. No. 04-12-1085) was pre- activated with 1.0 mmol of HBTU/HOBt and 2.0 mmol of DIEA for 3 minutes, and then it was allowed to react for 45 minutes on the resin. Deprotection was performed with 5.0 mL of 30% piperidine in DMF for 30 minutes, and spectrophotometric analysis gave a result of 189.0 µmol (yield 97.1%).

The two N-terminal cysteines were added in the form of Fmoc-L-Cys(Trt)-OH derivative (Novabiochem™, Laufelfingen, Switzerland, Cat. No. 04-13-1010); the amino acid activated with 1.0 mmol of HBTU/HOBt and 2.0 mmol of DIEA was added to the resin and the reaction mixture was kept for 1 hour under stirring at room temperature. Then incubation, deprotection of the Fmoc group and detection of the coupling yield were carried out as described above, obtaining a value of 187.3 µmol (yield 99.1%). The resin was washed 3 more times with 5.0 mL of DMF, 3 times with 5.0 mL of DCM, 3 times with 5.0 mL of methanol (MeOH, LabScan™, Dublin, Ireland, Cat. No. C2517), 3 times with 5.0 mL of diethyl ether (Et₂O, LabScan™, Dublin, Ireland, Cat. No. A3509E) and finally it was dried under vacuum. The weight of the dried resin was 363.5 mg; yield, 89.6% (theoretical 406.5 mg). To split the peptide from the resin and remove the protections on the side chains, the resin was treated for 2 hours at room temperature with 3.0 mL of a mixture consisting of:

| | |
|---|---|
| Reactant | % (w/w) |
| Trifluoroacetic acid (Sigma-Aldrich™, Milan, Italy, Cat. No. 91700) | 83 |
| MilliQ deionized H₂O | 4 |
| Phenol (Sigma-Aldrich™, Milan, Italy, Cat. No. 77612) | 6 |
| Thioanisole (Sigma-Aldrich™, Milan, Italy, Cat. No. 88470) | 5 |
| Tri-isopropylsilane (Sigma-Aldrich™, Milan, Italy, Cat. No. 92095) | 2 |

The resin was removed by filtration and the peptide was precipitated from the TFA solution by adding 20 ml of cold Et₂O. The precipitate was centrifuged and washed twice with cold Et₂O (2x10 mL), dissolved in 10 mL of H₂O/CH₃CN/TFA 50/50/0.1 (CH₃CN, acetonitrile, LabScan™, Dublin, Ireland, Cat. No. C2503) and, finally, lyophilized.

The weight of the lyophilized peptide was 109.5 mg, corresponding to a theoretical yield of raw product equal to 93.5% (calculated from the micromols of Fmoc splitted from the N-terminal cysteines).

Formation of the disulphide bridge (-S-S-) was carried out on a 20-mg aliquot of raw product. For this purpose, the product was dissolved in 200 mL of TRIS buffer solution (Tris(hydroxymethyl)aminomethane, Sigma-Aldrich™, Milan, Italy, Cat. No. 93347) 50 mM, pH 8.5 comprising 20% of dimethylsulphoxide (DMSO, LabScan™, Dublin, Ireland, Cat. No. H6534) and then stirred in an open 500-mL beaker for 48 hours under continuous stirring. Finally the solution was made acid with 5.0 M HCl (Sigma-Aldrich™, Milan, Italy, Cat. No. H1758) and concentrated in a rotary evaporator to a volume of approx. 100 mL. Purification of the peptide was carried out by preparative RP-HPLC using a column RP-18 25x2.2 cm ID (Phenomenex™, Torrance, CA) and an LC-8A chromatographic system (Shimadzu™, Milan, Italy), working at a flow rate of 20 mL/min. After equilibrating the column at 20 mL/min with 10% CH₃CN, 0.1% TFA, the solution of Compound IB was loaded onto the column and a gradient of CH₃CN, 0.1 % TFA from 10% to 60% was applied in 45 minutes. The outflow from the column was monitored at a wavelength of 210 nm. Compound IB was eluted at 24% of organic solvent and the fraction corresponding to the peak was lyophilized obtaining 10 mg. For the characterization of the purified product, a solution was prepared in H₂O/CH₃CN/TFA (50/50/0.1) at a concentration of 1.0 mg/mL and analysed with an LC-MS system (LCQ DUO, ThermoFinnigan™, Milan, Italy). The instrument was equipped with a complete HPLC system comprising pump, autosampler and photodiode detector and was coupled to a mass spectrometer provided with an electrospray source and ion trap analyser (ESI-IT). The HPLC was equipped with a LUNA™ column RP-18 30x2 mm ID, 3 µm operating at a flow rate of 200 µL/min. Detection was performed both by means of a photodiode detector operating in the wavelength range 200-400 nm, and using the mass spectrometer in the range of molecular weights between 200 and 2000 amu. During the analysis, the source was maintained at a voltage of 4.3 kV with a nitrogen flow of 20 Umin. After injection of 5 µL of solution, a gradient of CH₃CN, 0.1% TFA from 5% to 60% was applied in 11 minutes. Compound IB, in these analytical conditions, was eluted at a retention time of 5.4 minutes. Spectrometric analysis of the eluate was carried out by monitoring both positive ions and negative ions. The degree of purity of the product, deduced from the chromatogram extracted at a wavelength of 215 nm, was > 99%. The mass spectrum, extrapolated from the chromatographic run, gave a value for [M+H]⁺ of 1282.3 amu in positive mode and [M-H]⁻ of 1280.3 amu in negative mode, in excellent agreement with the theoretical value of 1281,5 amu.

The following Compounds I were prepared by working in a similar way:

(H₂N-L-Tyr-L-His-L-His-CO)₂-L-Lys-OH I(C)

(H₂N-L-Cys-D-Tyr-D-His-D-His-CO)₂-L-Lys-G ly-OH I(D)

(H₂N-L-Cys-D-His-D-His-D-Tyr-CO)₂-L-Lys-Gly-OH I(E)

(Ac-L-Tyr-L-His-L-His-CO)₂-L-Lys-Gly-OH I(F)

H₂N-L-Cys-L-His-L-His-L-Tyr-L-Ala-Gly-L-Ala-Gly-L-Ala-Gly-L-Ala-OH I(G)

(H₂N-L-Tyr-L-His-L-His-CO)₂-L-Lys-L-Lys-CO-L-Tyr-L-His-L-His-L-Cys-NH₂ I(H)

(H₂N-L-Cys-D-Tyr-D-His-D-His-CO)₄-L-Lys₂-L-Lys-Gly-OH I(J)

### EXAMPLE 2

### Immobilization of Compound IB on pre-activated resin Ultralink Biosupport Medium™

Compound IB (5 mg) prepared according to the preceding Example 1 was dissolved in 5.0 ml of 0.2 M NaHCO₃, 0.6 M Na citrate, pH 9.0, and incubated with 130 mg (1 mL) of resin Ultralink Biosupport Medium™ (PIERCE, Rockford, IL, USA), a support for affinity chromatography pre-activated with a function of the azalactone type for direct coupling of peptides and proteins. The suspension was stirred for 12 hours at room temperature and the extent of coupling was monitored by taking samples of the reaction mixture at various times and then analysing by RP-HPLC.

After 12 hours, approx. 90-95% of the initial peptide had been immobilized covalently on the resin. The derivatized resin was incubated with 0.1 M TRIS, pH 8.5 to block any sites of the resin that are still reactive, and then packed in a glass column 100 x 0.6 mm ID with variable plunger (OMNIFIT™, Cat. No. 006CC-06-10-FF).

The following Compounds I were immobilized in a similar manner on pre-activated resin Ultralink Biosupport Medium™:

(H₂N-L-Tyr-L-His-L-His-CO)₂-L-Lys-OH I(C)

(H₂N-L-Cys-D-His-D-His-D-Tyr-CO)₂-L-Lys-Gly-OH I(E)

### EXAMPLE 3

### Separation and purification of immunoglobulins from human and murine serum by affinity chromatography

To evaluate the capability of Compound I(B) immobilized on resin according to Example 2 to separate and purify immunoglobulins, the column was equilibrated with buffer 25 mM Bis-Tris (Bis(2-hydroxyethyl)amino-tris(hydroxymethyl)methane, Sigma-Aldrich™, Cat. No. B7535), pH 6.5, at a flow rate of 1 mL/min, monitoring the eluent at 280 nm.

Raw human serum (Sigma-Aldrich™, Cat. No. R9133) was filtered, diluted 1:1 (v/v) with the binding buffer, loaded on the column and, after elution of the unbound material, the eluent was changed to acetic acid (AcH, Sigma-Aldrich™, Cat. No. 46928) 0.1 M, pH 3.0, for eluting the adsorbed material.

The material eluted from the column was neutralized with TRIS 1 M, pH 9.0 and characterized by analysis by polyacrylamide gel electrophoresis and Western blotting (WB) on nitrocellulose for identifying the immunoglobulins retained.

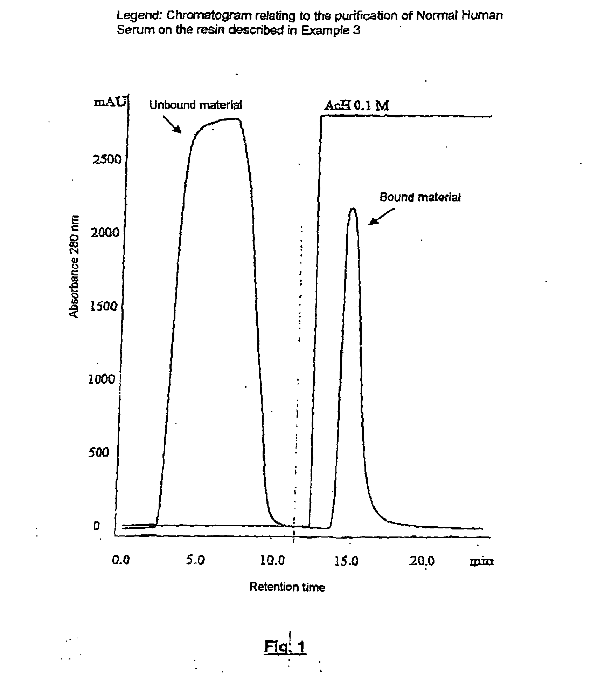

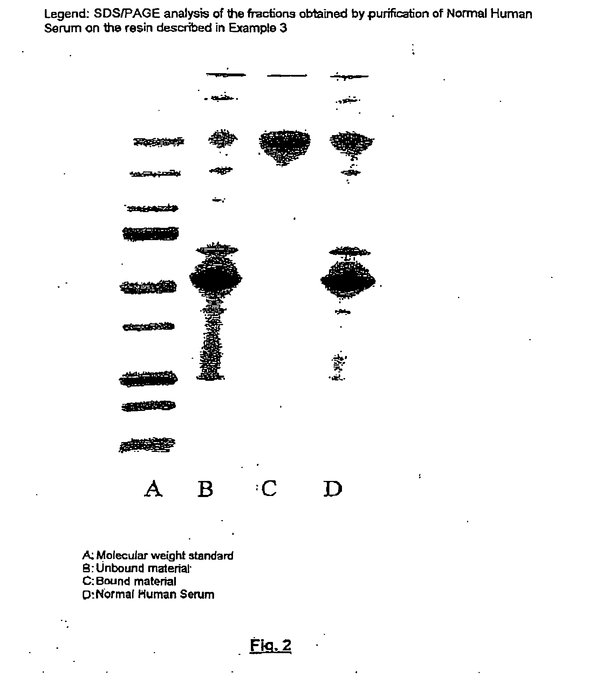

The separation and purification of immunoglobulins from human serum by affinity chromatography is shown in Fig. 1, whereas Fig. 2 shows the electrophoretic analysis of the fractions collected during the process.

As is clearly shown by the electrophoretic analysis, the column was able to retain the immunoglobulin fraction of the raw serum, whereas the albumin was recovered in the unretained material.

WB analysis of the retained material revealed that the column retained all the classes of immunoglobulins (IgG, IgE, IgM and IgA) present in human serum.

Similar results were obtained when using the raw murine serum (Sigma-Aldrich™, Cat. No. 5905). In this case too, the column was able to retain the IgG, IgE, IgM and IgA immunoglobulins, whereas the albumin was recovered in the unretained material.

Similar results were obtained with the following Compounds I:

(H₂N-L-Tyr-L-His-L-His-CO)₂-L-Lys-OH I(C)

(H₂N-L-Cys-D-His-D-His-D-Tyr-CO)₂-L-Lys-Gly-OH I(E)

## Claims

1. A compound of formula:
(X₁-X₂-His-X₃-CO)ₙ-R (I)
where:
X₁ is H or an amino acid residue of cysteine (Cys) having L or D configuration, wherein each pair of amino acid residues of Cys, optionally present as X₁, can be in the form of a cystine residue owing to an -S-S- bridge formed by the -SH functions of two Cys residues,
X₂ is an amino acid residue of tyrosine (Tyr) or of histidine (His) having L or D configuration,
X₃ is an amino acid residue of His or Tyr having L or D configuration, provided, however, that X₂ is Tyr when X₃ is His and X₂ is His when X₃ is Tyr,
His is an amino acid residue of histidine having L or D configuration, n is an integer of from 2 to 4,
R is a group capable of forming a dimer, a trimer or a tetramer, comprising from 1 to 3 amino acid residues of Lys.

2. A compound according to Claim 1, **characterized in that** the N-terminal group or groups (NH₂) belonging to X₁ when X₁ is Cys or to X₂ when X₁ is H are acylated by acylating groups capable of neutralizing their nucleophilicity/basicity and/or of immobilizing said Compound non-covalently on a solid support wherein the acylating group is selected from the group comprising:
- natural amino acids selected from the group comprising L-tryptophan, D-tryptophan, L-tyrosine, D-tyrosine;
- synthetic amino acids selected from the group comprising L-naphthylalanine, cyclohexylalanine, aminonorbornyl carboxylate, L-N-methyltryptophan, L-N-methyltyrosine, N-methyl-α-naphthylalanine, D-N-methyltryptophan, D-N-methoyltyrosine, N-benzylglycine, N-(3-indolylethyl)glycine, N-(p-hydroxyphenylethyl)glycine, N-cyclopropyl-glycine, N-cyclobutyl-glycine, N-cyclo-hexyl-glycine, N-cyclo-opta-glycine, N-cyclooctylglycine, N-cyclodecylglycine, N-cycloundecylglycines, N-cyclododecylglycines, N-(2,2-diphenyl-ethyl)glycine, N-(3,3-diphenyl-propyl)glycine, N-(N-(2,2-diphenyl-ethyl)carbamyl-methyl)-glycine, N-(N-(3,3-diphenyl-propyl)carbamylmethyl)glycine, 1-carboxy-1-(2,2-diphenyl-ethylamino)-cyclopropane;
- carboxylic acids and derivatives thereof selected from the group comprising naphthylacetic, benzoic, 4-methoxybenzoic, 2,4-dichlorobenzoic, 2-nitrobenzoic, 4-chlorobenzoic, phenylacetic, diphenylacetic, (R)-2-phenylpropanoic, (S)-2-phenylpropanoic, (S)-3-phenylpropanoic, (R)-3-phenylpropanoic, (R,S)-2-methyl,3-phenylbutanoic, (S,R)-2-methyl,3-phenylbutanoic, (R,R)-2-methyl,3-phenylbutanoic, (S,S)-2-methyl,3-phenylbutanoic, (R,S)-2,3-diphenylpentanoic, (R,R)-2,3-diphenylpentanoic, (S,S)-2,3-diphenylpentanoic, (S,R)-2,3-diphenylpentanoic, 9-fluorenyl-methoxycarponyl chloride, carbobenzyloxy chloride, 2-chloro carbobenzyloxychloride.

3. A compound according to Claim 2, **characterized in that** the group capable of immobilizing a peptide non-covalently on a solid support is selected from the group comprising fluorenyl-methoxycarbonyl (Fmoc) and carboxybenzyl (Cbz).

4. A compound according to Claim 3, **characterized in that** the solid support is selected from the group comprising synthetic polymers.

5. Compound according to Claim 4, **characterized in that** the synthetic polymer is selected from the group comprising PVC, polystyrene and polyethylene.

6. A compound according to any one of Claims from 1 to 5, **characterized in that** the group R is bound to a molecule selected from the group comprising biotin and fluorescein.

7. A compound according to any one of the preceding Claims from 1 to 6, **characterized in that** the group R is bound to a group of formula:
(H₂N-Cys-Tyr-His-His-CO)₂-Lys-Gly₃-Lys-(ε-Cbz)-COOH (IA)

8. A compound according to Claim 7, **characterized in that** the pair of Cys amino acid residues is in the form of a cystine residue owing to an -S-S- bridge formed by the -SH functions of two Cys residues.

9. A compound according to Claim 7 or 8, **characterized in that** an N-terminal group (NH₂) is acylated by an acylating group according to any one of claims 2 and 3. neutralizing its nucleophilicity and/or basicity or of immobilizing a peptide on a solid support.

10. A compound according to Claim 7 or 8, **characterized in that** both N-terminal groups (NH₂) are acylated by acylating groups according to any one of claims 2 and 3.

11. A method of solid-phase preparation of a Compound according to any one of Claims from 1 to 10, **characterized in that** it comprises the following steps:
a) coupling of a first amino acid in D or L configuration to a labile acidic support for solid-phase peptide synthesis; the functional group of said first amino acid used for anchoring to the resin being such that it can be hydrolysed by treatment with strong acids such as trifluoroacetic acid (TFA), whereas the second functional group is provided with suitable protection that can be removed after anchoring to the resin; if the first amino acid is trifunctional, for example Cys or Lys, two functions will be protected during anchoring to the resin;
b) optional addition of groups such as biotin or fluorescein on a side group;
c) optional sequential addition of 2-7 amino acid residues, still according to the protection-coupling-deprotection technique, said amino acid groups preferably comprising glycine and/or D- or L-alanine;
d) subsequent sequential coupling of 1 (dime), 2 (trimer) or 3 (tetramer) residues of D- or L-lysine protected at their α and ε amino functional groups with the same group such as the group Fmoc,
e) sequential coupling of suitable amino acid residues such as D- or L-histidine protected at the N^{τ} imidazole group with a suitable group such as triphenylmethyl (trityl or Trt), or D- or L-tyrosine protected at the phenolic group with a suitable group such as tert-butyl (tBu); a D- or L-histidine with similar Trt protection; a residue of D- or L-histidine protected at the N^{τ} imidazole group with a Trt group, or D- or L-tyrosine protected at the phenolic group with a tBu group; finally a residue of D- or L-cysteine provided with Trt protection on the thyol function; all the amino acid residues also being suitably protected on the α amino function by a suitable removable protecting group such as Fmoc;
f) addition to the N-terminal functions of the last amino acid; of optional protecting groups such as acetyl groups, Fmoc, Cbz;
g) splitting of the various protecting groups of the peptide with the exclusion, optionally, of those on the N-terminal groups and removal thereof from the resin, and
h) optional cyclization of the peptide by formation of a disulphide bridge between the cysteine pairs optionally present on said peptide molecule.

12. A method of separating and purifying at least one immunoglobulin that comprises the following steps:
(i) immobilization on a support for affinity chromatography of a compound that is able to bind non-covalently at least one immunoglobulin,
(ii) packing of said support for affinity chromatography in a chromatographic column,
(iii) flushing of said column with a buffer capable of promoting the formation of bonds between immunoglobulin and the immobilized compound,
(iv) loading of said column with a fluid comprising at least one immunoglobulin,
(v) washing of said column with a liquid capable of eluting the impurities without interfering with the bond between the immunoglobulin and the immobilized compound,
(vi) elution of the immunoglobulin adsorbed on the column with a dissociating eluent,
and is **characterized in that:**
(vii) the compound that is capable of binding non-covalently at least one immunoglobulin is a Compound according to one of the preceding Claims from 1 to 10.

13. A method according to Claim 12, **characterized in that** the support for affinity chromatography is of the type pre-activated with epoxy, azalactone or ester group for the direct coupling of peptides and proteins.

14. A method according to Claim 12 or 13, **characterized in that** step (i) is carried out in the presence of a basic buffer solution, the pH of which is between 8.5 and 9.5, and does not comprise compounds provided with free amino functions or other nucleophilic groups.

15. A method according to any one of Claims from 12 to 14, **characterized in that** step (iii) is carried out with a neutral buffer solution selected from the group comprising a solution of Bis-Tris, pH 6.5, and a solution of sodium phosphate, pH 7.0.

16. A method according to any one of Claims from 12 to 15, **characterized in that** step (v) is carried out with a neutral buffer solution of low ionic strength selected from the group comprising a solution of Bis-Tris 25 mM, pH 6.5, and a solution of sodium phosphate, 50 mM, pH 7.0.

17. A method according to any one of Claims from 12 to 16, **characterized in that** a dissociating eluent consisting of an acidic or basic aqueous solution selected from the group comprising an aqueous solution of acetic acid 0.1 M pH 3.0, and of sodium bicarbonate 100 mM pH 9.0, is used in step (v).

18. A method for detecting an immunoglobulin or a mixture of immunoglobulins by the ELISA technique comprising the following steps:
(1) immobilization on a microplate for ELISA analysis of a compound capable of binding non-covalently at least one immunoglobulin,
(2) incubation, on the microplate, of a sample comprising the immunoglobulin or immunoglobulins of interest,
(3) detection of the complex formed from the immobilized compound/immunoglobulin,
and is **characterized in that:**
(4) the compound capable of binding at least one immunoglobulin is a Compound of formula (I) according to any one of the preceding Claims from 1 to 10.

## Patentansprüche

1. Verbindung der Formel:
(X₁-X₂-His-X₃-CO)ₙ-R (1)
worin:
X₁ H oder ein Aminosäurerest von Cystein (Cys) in der L- oder D-Konfiguration ist, worin jedes Paar von Aminosäureresten von Cys, das gegebenenfalls als X₁ vorliegt, in der Form eines Cystinrestes infolge einer -S-S-Brücke sein kann, die durch die -SH-Funktionen zweier Cys-Reste gebildet wird,
X₂ ein Aminosäurerest von Tyrosin (Tyr) oder von Histidin (His) in der L- oder D-Konfiguration ist,
X₃ ein Aminosäurerest von His oder Tyr in der L- oder D-Konfiguration ist, vorausgesetzt jedoch, daß X₂ Tyr ist wenn X₃ His ist und X₂ His ist wenn X₃ Tyr ist,
His ein Aminosäurerest von Histidin in der L- oder D-Konfiguration ist,
n eine ganze Zahl von 2 bis 4 ist,
R eine Gruppe ist, die ein Dimer, ein Trimer oder ein Tetramer bilden kann, umfassend von 1 bis 3 Aminosäurereste von Lys.

2. Verbindung gemäß Anspruch 1, **dadurch gekennzeichnet, daß** die N-terminale(n) Gruppe oder Gruppen (NH₂), die zu X₁ gehören, wenn X₁ Cys ist, oder zu X₂ gehören, wenn X₁ H ist, durch acylierende Gruppen acyliert sind, die ihre Nukleophilie/Basizität neutralisieren und/oder die Verbindung nicht-kovalent an einem Trägermaterial immobilisieren können, worin die acylierende Gruppe aus der Gruppe ausgewählt ist, umfassend:
- natürliche Aminosäuren, die aus der Gruppe ausgewählt sind, die L-Tryptophan, D-Tryptophan, L-Tyrosin und D-Tyrosin umfaßt;
- synthetische Aminosäuren, die aus der Gruppe ausgewählt sind, die L-Naphthylalanin, Cyclohexylalanin, Aminonorbornylcarboxylat, L-N-Methyltryptophan, L-N-Methyltyrosin, N-Methyl-α-naphthylalanin, D-N-Methyltryptophan, D-N-Methoyltyrosin, N-Benzylglycin, N-(3-Indolylethyl)glycin, N-(p-Hydroxyphenylethyl)glycin, N-Cyclopropylglycin, N-Cyclobutyl-glycin, N-Cyclohexyl-glycin, N-Cycloopta-glycin, N-Cyclooctyl-glycin, N-Cyclodecyl-glycin, N-Cycloundecyl-glycine, N-Cyclododecyl-glycine, N-(2,2-Diphenyl-ethyl)glycin, N-(3,3-diphenyl-propyl)glycin, N-(N-(2,2-Diphenyl-ethyl)carbamyl-methyl)glycin, N-(N-(3,3-Diphenyl-propyl)carbamyl-methyl)glycin und 1-Carboxy-1-(2,2-diphenylethylamino)cyclopropan umfaßt;
- Carbonsäuren und Derivate davon, die aus der Gruppe ausgewählt sind, die aus Naphthylessigsäure, Benzoesäure, 4-Methoxybenzoesäure, 2,4-Dichlorbenzoesäure, 2-Nitrobenzoesäure, 4-Chlorbenzoesäure, Phenylessigsäure, Diphenylessigsäure, (R)-2-Phenylpropansäure, (S)-2-Phenylpropansäure, (S)-3-Phenylpropansäure, (R)-3-Phenylpropansäure, (R,S)-2-Methyl-3-phenylbuttersäure, (S,R)-2-Methyl-3-phenylbuttersäure, (R,R)-2-Methyl-3-phenylbuttersäure, (S,S)-2-Methyl-3-phenylbuttersäure, (R,S)-2,3-Diphenylpentansäure, (R,R)-2,3-Diphenylpentansäure, (S,S)-2,3-Diphenylpentansäure, (S,R)-2,3-Diphenylpentansäure, 9-Fluorenyl-methoxycarbonylchlorid, Carbobenzyloxychlorid und 2-Chlorcarbobenzyloxychlorid umfaßt.

3. Verbindung gemäß Anspruch 2, **dadurch gekennzeichnet, daß** die Gruppe, die ein Peptid nicht-kovalent an einem Trägermaterial immobilisieren kann, aus der Gruppe ausgewählt ist, die Fluorenyl-methoxycarbonyl (Fmoc) und Carboxybenzyl (Cbz) umfaßt.

4. Verbindung gemäß Anspruch 3, **dadurch gekennzeichnet, daß** das Trägermaterial aus der Gruppe ausgewählt ist, die synthetische Polymere umfaßt.

5. Verbindung gemäß Anspruch 4, **dadurch gekennzeichnet, daß** das synthetische Polymer aus der Gruppe ausgewählt ist, die PVC, Polystyrol und Polyethylen umfaßt.

6. Verbindung gemäß einem der Ansprüche 1 bis 5, **dadurch gekennzeichnet, daß** die Gruppe R an ein Molekül gebunden ist, das aus der Gruppe ausgewählt ist, die Biotin und Fluorescein umfaßt.

7. Verbindung gemäß einem der vorangegangenen Ansprüche 1 bis 6, **dadurch gekennzeichnet, daß** die Gruppe R an eine Gruppe der Formel:
(H₂N-Cys-Tyr-His-His-CO)₂-Lys-Gly₃-Lys-(ε-Cbz)-COOH (IA)
gebunden ist.

8. Verbindung gemäß Anspruch 7, **dadurch gekennzeichnet, daß** das Paar von Cys-Aminosäureresten infolge einer -S-S-Brücke, die durch die -SH-Funktionen von zwei Cys-Resten gebildet wird, in der Form eines Cystinrestes ist.

9. Verbindung gemäß Anspruch 7 oder 8, **dadurch gekennzeichnet, daß** eine N-terminale Gruppe (NH₂) durch eine acylierende Gruppe gemäß einem der Ansprüche 2 und 3 acyliert ist.

10. Verbindung gemäß Anspruch 7 oder 8, **dadurch gekennzeichnet, daß** beide N-terminalen Gruppen (NH₂) durch acylierende Gruppen gemäß einem der Ansprüche 2 und 3 acyliert sind.

11. Verfahren zur Festphasen-Herstellung einer Verbindung gemäß einem der Ansprüche 1 bis 10, **dadurch gekennzeichnet, daß** es die folgenden Schritte umfaßt:
a) Koppeln einer ersten Aminosäure in D- oder L-Konfiguration an einen labilen, sauren Träger für die Festphasen-Peptidsynthese; die funktionelle Gruppe der ersten Aminosäure, die für das Verankern an das Harz verwendet wird, derart ist, daß sie durch Behandlung mit starken Säuren, wie Trifluoressigsäure (TFA), hydrolysiert werden kann, während die zweite funktionelle Gruppe mit einem geeigneten Schutz versehen ist, der nach dem Verankern an das Harz entfernt werden kann; sofern die erste Aminosäure trifunktionelle ist, z.B. Cys oder Lys, werden zwei Funktionen während des Verankerns an das Harz geschützt sein;
b) optionales Hinzufügen von Gruppen, wie Biotin oder Fluorescein, an eine Seitengruppe;
c) optionales sequentielles Hinzufügen von 2 bis 7 Aminosäureresten, nach wie vor gemäß der Schützen-Koppeln-Entschützen-Technik, wobei die Aminosäuregruppen vorzugsweise Glycin und/oder D- oder L-Alanin umfassen;
d) anschließendes sequentielles Koppeln von 1 (Dimer), 2 (Trimer) oder 3 (Tetramer) Resten von D- oder L-Lysin, die an ihren funktionellen α- und ε-Aminogruppen mit der gleichen Gruppe, wie die Gruppe Fmoc, geschützt sind;
e) sequentielles Koppeln von geeigneten Aminosäureresten, wie D- oder L-Histidin, das an seiner N^{τ}-Imidazolgruppe mit einer geeigneten Gruppe, wie Triphenylmethyl (Trityl oder Trt), geschützt ist, oder D- oder L-Tyrosin, das an der Phenolgruppe mit einer geeigneten Gruppe, wie tert-Butyl (tBu), geschützt ist; ein D- oder L-Histidin mit gleichem Trt-Schutz; ein Rest von D- oder L-Histidin, der an der N^{τ}-Imidazolgruppe mit einer Trt-Gruppe geschützt ist, oder D- oder L-Tyrosin, das an der Phenolgruppe mit einer tBu-Gruppe geschützt ist; schließlich ein Rest von D- oder L-Cystein, das mit einem Trt-Schutz auf der Thyol-Funktion versehen ist; wobei alle Aminosäurereste an der α-Aminofunktion durch eine geeignete entfernbare Schutzgruppe, wie Fmoc, geeigneterweise ebenfalls geschützt sind;
f) Hinzufügen von optionalen Schutzgruppen, wie Acetylgruppen, Fmoc oder Cbz, an die N-terminalen Funktionen der letzten Aminosäure;
g) Abspalten der verschiedenen Schutzgruppen vom Peptid, mit der Ausnahme von gegebenenfalls denjenigen an den N-terminalen Gruppen, und Entfernen dieser vom Harz; und
h) optionales Zyklisieren des Peptids durch Bildung einer Disulfidbrücke zwischen den Cysteinpaaren, die gegebenenfalls im Peptidmolekül vorliegen.

12. Verfahren zur Trennung und Aufreinigung wenigstens eines Immunglobulins, das die folgenden Schritte umfaßt:
(i) Immobilisieren einer Verbindung, die in der Lage ist, wenigstens ein Immunglobulin nicht-kovalent zu binden, an einen Träger für die Affinitätschromatographie,
(ii) Packen des Trägers für die Affinitätschromatographie in eine Chromatographiesäule,
(iii) Spülen der Säule mit einem Puffer, der die Bildung von Bindungen zwischen dem Immunglobulin und der immobilisierten Verbindung begünstigen kann,
(iv) Beladen der Säule mit einer Flüssigkeit, die wenigstens ein Immunglobulin umfaßt,
(v) Waschen der Säule mit einer Flüssigkeit, die die Unreinheiten ohne Stören der Bindung zwischen dem Immunglobulin und der immobilisierten Verbindung eluieren kann,
(vi) Eluieren des Immunglobulins, das auf der Säule adsorbiert ist, mit einem dissoziierenden Elutionsmittel,
und das **dadurch gekennzeichnet ist, daß**
(vii) die Verbindung, die wenigstens ein Immunglobulin nicht-kovalent binden kann, eine Verbindung gemäß einem der vorangegangenen Ansprüche 1 bis 10 ist.

13. Verfahren gemäß Anspruch 12, **dadurch gekennzeichnet, daß** der Träger für die Affinitätschromatographie einer vom mit Epoxid-, Azalacton- oder Estergruppe voraktivierten Typ für die Direktkopplung von Peptiden und Proteinen ist.

14. Verfahren gemäß Anspruch 12 oder 13, **dadurch gekennzeichnet, daß** Schritt (i) in Gegenwart einer basischen Pufferlösung durchgeführt wird, deren pH zwischen 8,5 und 9,5 liegt und die keine Verbindungen umfaßt, die mit freien Aminofunktionen oder anderen nukleophilen Gruppen versehen sind.

15. Verfahren gemäß einem der Ansprüche 12 bis 14, **dadurch gekennzeichnet, daß** Schritt (iii) mit einer neutralen Pufferlösung durchgeführt wird, die aus der Gruppe ausgewählt ist, die eine Lösung von Bis-Tris, pH 6,5, und eine Lösung von Natriumphosphat, pH 7,0, umfaßt.

16. Verfahren gemäß einem der Ansprüche 12 bis 15, **dadurch gekennzeichnet, daß** Schritt (v) mit einer neutralen Pufferlösung von niedriger Ionenstärke durchgeführt wird, die aus der Gruppe ausgewählt ist, die eine Lösung von Bis-Tris 25 mM, pH 6,5, und eine Lösung von Natriumphosphat 50 mM, pH 7,0, umfaßt.

17. Verfahren gemäß einem der Ansprüche 12 bis 16, **dadurch gekennzeichnet, daß** ein dissoziierendes Elutionsmittel in Schritt (v) verwendet wird, das aus einer sauren oder basischen wäßrigen Lösung besteht, die aus der Gruppe ausgewählt ist, die eine wäßrige Lösung von Essigsäure 0,1 M, pH 3,0, und von Natriumbicarbonat 100 mM, pH 9,0, umfaßt.

18. Verfahren zum Feststellen eines Immunglobulins oder eines Gemisches von Immunglobulinen durch die ELISA-Technik, umfassend die folgenden Schritte:
(1) Immobilisierung einer Verbindung, die wenigstens ein Immunglobulin nicht-kovalent binden kann, an eine Mikrotiterplatte für ELISA-Analyse,
(2) Inkubation einer Probe, die das Immunglobulin oder die Immunglobuline von Interesse umfaßt, auf der Mikrotiterplatte,
(3) Feststellen des Komplexes, der durch die immobilisierte Verbindung/das Immunglobulin gebildet wurde,
und das **dadurch gekennzeichnet ist, daß**
(4) die Verbindung, die wenigstens ein Immunglobulin binden kann, eine Verbindung der Formel (I) gemäß einem der vorangegangenen Ansprüche 1 bis 10 ist.

## Revendications

1. Composé de formule :
(X₁-X₂-His-X₃-CO)ₙ-R (I)
où :
X₁ est H ou un résidu d'acide aminé de cystéine (Cys) ayant une configuration L ou D, dans lequel chaque paire de résidus d'acides aminés de Cys, facultativement présente en tant que X₁, peut être sous la forme d'un résidu de cystine appartenant à un pont -S-S- formé par les fonctions -SH de deux résidus Cys,
X₂ est un résidu d'acide aminé de tyrosine (Tyr) ou d'histidine (His) ayant une configuration L ou D,
X₃ est un résidu d'acide aminé de His ou Tyr ayant une configuration L ou D, à condition, toutefois, que X₂ soit Tyr lorsque X₃ est His et X₂ soit His lorsque X₃ est Tyr,
His est un résidu d'acide aminé d'histidine ayant une configuration L ou D,
n est un nombre entier de 2 à 4,
R est un groupe capable de former un dimère, un trimère ou un tétramère, comprenant de 1 à 3 résidus d'acides aminés de Lys.

2. Composé selon la revendication 1, **caractérisé en ce que** le groupe ou les groupes N-terminaux (NH₂) appartenant à X₁ lorsque X₁ est Cis ou à X₂ lorsque X₁ est H sont acylés en acylant des groupes capables de neutraliser leur nucléophilie/basicité et/ou d'immobiliser ledit composé de manière non covalente sur un support solide, dans lequel le groupe d'acylation est choisi dans le groupe comprenant :
- des acides aminés naturels choisis dans le groupe comprenant le L-tryptophane, le D-tryptophane, la L-tyrosine, la D-tyrosine ;
- des acides aminés synthétiques choisis dans le groupe comprenant la L-naphtylalanine, la cyclohexylalanine, le carboxylate d'aminonorbornyle, le L-N-méthyltryptophane, la L-N-méthyltyrosine, la N-méthyl-α-naphtylalanine, le D-N-méthyltryptophane, la D-N-méthoyltyrosine, la N-benzyglycine, la N-(3-indolyléthyl)glycine, la N-(p-hydroxyphényl-éthyl)glycine, la N-cyclopropyl-glycine, la N-cyclobutyl-glycine, la N-cyclo-hexyl-glycine, la N-cyclo-opta-glycine, la N-cyclooctylglycine, la N-cyclodécylglycine, les N-cycloundécylglycines, les N-cyclododécylglycines, la N-(2,2-diphényl-éthyl)glycine, la N-(3,3-diphényl-propyl)glycine, la N-(N-(2,2-diphényl-éthyl)carbamyl-méthyl)-glycine, la N-(N-(3,3-diphényl-propyl)carbamyl-méthyl)glycine, le 1-carboxy-1-(2,2-diphényl-éthylamino)-cyclopropane ;
- des acides carboxyliques et leurs dérivés choisis dans le groupe comprenant les acides naphtylacétique, benzoïque, 4-méthoxybenzoïque, 2,4-dichlorobenzoïque, 2-nitrobenzoïque, 4-chlorobenzoïque, phénylacétique, diphénylacétique, (R)-2-phénylpropanoïque, (S)-2-phénylpropanoïque, (S)-3-phénylpropanoïque, (R)-3-phénylpropanoïque, (R,S)-2-méthyl,3-phénylbutanoïque, (S,R)-2-méthyl,3-phénylbutanoïque, (R,R)-2-méthyl,3-phénylbutanoïque, (S,S)-2-méthyl,3-phénylbutanoïque, (R,S)-2,3-diphénylpentanoïque, (R,R)-2,3-diphénylpentanoïque, (S,S)-2,3-diphénylpentanoïque, (S,R)-2,3-diphénylpentanoïque, le chlorure de 9-fluorényl-méthoxycarbonyle, le chlorure de carbobenzyloxy, le chlorure de 2-chloro carbobenzyloxy.

3. Composé selon la revendication 2, **caractérisé en ce que** le groupe capable d'immobiliser un peptide de manière non covalente sur un support solide est choisi dans le groupe comprenant le fluorényl-méthoxycarbonyle (Fmoc) et le carboxybenzyle (Cbz).

4. Composé selon la revendication 3, **caractérisé en ce que** le support solide est choisi dans le groupe comprenant les polymères synthétiques.

5. Composé selon la revendication 4, **caractérisé en ce que** le polymère synthétique est choisi dans le groupe comprenant le PVC, le poly(styrène) et le poly(éthylène).

6. Composé selon l'une quelconque des revendications 1 à 5, **caractérisé en ce que** le groupe R est lié à une molécule.

7. Composé selon l'une quelconque des revendications 1 à 6 précédentes, **caractérisé en ce que** le groupe R est lié à un groupe de formule
(H₂N-Cys-Tyr-His-His-CO)₂-Lys-Gly₃-Lys-(ε-Cbz)-COOH (IA)

8. Composé selon la revendication 7, **caractérisé en ce que** la paire de résidus d'acides aminés Cys est sous la forme d'un résidu de cystine appartenant à un pont -S-S- formé par les fonctions -SH de deux résidus Cys.

9. Composé selon la revendication 7 ou 8, **caractérisé en ce qu'**un groupe N-terminal (NH₂) est acylé par un groupe d'acylation selon l'une quelconque des revendications 2 et 3.

10. Composé selon la revendication 7 ou 8, **caractérisé en ce que** les deux groupes N-terminaux (NH₂) sont acylés par des groupes d'acylation selon l'une quelconque des revendications 2 et 3.

11. Procédé de préparation en phase solide d'un composé selon l'une quelconque des revendications 1 à 10, **caractérisé en ce qu'**il comprend les étapes suivantes :
a) couplage d'un premier acide aminé de configuration D ou L à un support acide labile pour une synthèse de peptide en phase solide ; le groupe fonctionnel dudit premier acide aminé utilisé pour ancrage à la résine étant tel qu'il peut être hydrolysé par traitement avec des acides forts tels que l'acide trifluoroacétique (TFA), tandis que le second groupe fonctionnel est pourvu d'une protection appropriée qui peut être éliminée après ancrage à la résine ; si le premier acide aminé est trifonctionnel, par exemple Cys ou Lys, deux fonctions seront protégées pendant l'ancrage à la résine ;
b) addition facultative de groupes tels que biotine ou fluorescéine sur un groupe latéral ;
c) addition séquentielle facultative de 2 à 7 résidus d'acides aminés, toujours selon la technique de protection-couplage-déprotection, lesdits groupes acides aminés comprenant de préférence de la glycine et/ou de la D- ou L-alanine ;
d) couplage séquentiel ultérieur de 1 (dimère), 2 (trimère) ou 3 (tétramère) résidus de D- ou L-lysine protégés au niveau de leurs groupes α et ε-amino fonctionnels avec le même groupe tel que le groupe Fmoc,
e) couplage séquentiel de résidus d'acides aminés appropriés tels que D- ou L-histidine protégée au niveau du groupe N⁺ imidazole avec un groupe approprié tel que le triphénylméthyle (trityle ou Trt) ou D- ou L-tyrosine protégée au niveau du groupe phénolique avec un groupe approprié tel que tert-butyle (tBu) ; une D- ou L-histidine avec une protection Trt similaire ; un résidu de D- ou L-histidine protégée au niveau du groupe N⁺ imidazole avec un groupe Trt, ou D- ou L-tyrosine protégée au niveau du groupe phénolique avec un groupe tBu ; enfin un résidu de D- ou L-cystéine pourvu d'une protection Trt sur la fonction thiol ; tous les résidus d'acides aminés étant également protégés de manière appropriée sur la fonction α-amino par un groupe protecteur pouvant être éliminé approprié tel que Fmoc ;
f) addition aux fonctions N-terminales du dernier acide aminé, des groupes protecteurs facultatifs tels que des groupes acétyle, Fmoc, Cbz ;
g) séparation des divers groupes protecteurs du peptide à l'exclusion facultative de ceux sur les groupes N-terminaux et leur élimination de la résine, et
h) cyclisation facultative du peptide par formation d'un pont disulfure entre les paires de cystéines facultativement présentes sur ladite molécule peptidique.

12. Procédé de séparation et de purification d'au moins une immunoglobuline qui comprend les étapes suivantes :
(i) immobilisation sur un support pour chromatographie d'affinité d'un composé qui est capable de se lier de manière non covalente à au moins une immunoglobuline,
(ii) garnissage du support pour chromatographie d'affinité dans une colonne chromatographique,
(iii) purge de ladite colonne avec un tampon capable de favoriser la formation de liaisons entre une immunoglobuline et le composé immobilisé,
(iv) chargement de ladite colonne avec un fluide comprenant au moins une immunoglobuline,
(v)lavage de ladite colonne avec un liquide capable d'éluer les impuretés sans interférer avec la liaison entre l'immunoglobuline et le composé immobilisé,
(vi) élution de l'immunoglobuline adsorbée sur la colonne avec éluant de dissociation,
et est **caractérisé en ce que :**
(vii) le composé qui est capable de se lier de manière non covalente à au moins une immunoglobuline est un composé selon l'une quelconque des revendications 1 à 10 précédentes.

13. Procédé selon la revendication 12, **caractérisé en ce que** le support pour chromatographie d'affinité est du type préactivé avec un groupe époxy, azalactone ou ester pour le couplage direct de peptides et de protéines.

14. Procédé selon la revendication 12 ou 13, **caractérisé en ce que** l'étape (i) est réalisée en présence d'une solution tampon basique, dont le pH est compris entre 8,5 et 9,5, et ne comprend pas de composés pourvus de fonctions amino libres ou d'autres groupes nucléophiles.

15. Procédé selon l'une quelconque des revendications 12 à 14, **caractérisé en ce que** l'étape (iii) est réalisée avec une solution tampon neutre choisie dans le groupe comprenant une solution de Bis-Tris, pH 6,5, et une solution de phosphate de sodium, pH 7,0.

16. Procédé selon l'une quelconque des revendications 12 à 15, **caractérisé en ce que** l'étape (v) est réalisée avec une solution tampon neutre choisie de faible force ionique dans le groupe comprenant une solution de Bis-Tris à 25 mM, pH 6,5, et une solution de phosphate de sodium à 50 mM, pH 7,0.

17. Procédé selon l'une quelconque des revendications 12 à 16, **caractérisé en ce qu'**un éluant de dissociation consistant en une solution aqueuse acide ou basique choisie dans le groupe comprenant une solution aqueuse d'acide acétique à 0,1 M, pH 3,0 et de bicarbonate de sodium à 100 mM, pH 9,0, est utilisé dans l'étape (v).

18. Procédé de détection d'une immunoglobuline ou d'un mélange d'immunoglobulines par la technique ELISA comprenant les étapes suivantes :
(1) immobilisation sur une microplaque pour analyse ELISA d'un composé capable de se lier de manière covalente à au moins une immunoglobuline,
(2) incubation, sur la microplaque, d'un échantillon comprenant l'immunoglobuline ou les immunoglobulines d'intérêt,
(3) détection du complexe formé à partir du composé/de l'immunoglobuline immobilisé,
et est **caractérisé en ce que :**
(4) le composé capable de se lier à au moins une immunoglobuline est un composé de formule (I) selon l'une quelconque des revendications 1 à 10 précédentes.
